# EUROPÄISCHE PATENTSCHRIFT

(11) **EP 0 461 563 B1**
(45) Veröffentlichungstag und Bekanntmachung des Hinweises auf die Patenterteilung: **27.04.1994**
(21) Anmeldenummer: 91109393.8
(22) Anmeldetag: 07.06.1991
(51) Int. Cl.: C07C 17/20, C07C 19/08

(54) **Verfahren zur Herstellung von weitgehend fluorierten Alkylbromiden**
Process for the preparation of highly fluorinated alkyl bromides
Procédé pour la préparation de bromures d'alkyle fortement fluorés

(30) Priorität: 13.06.1990 DE 4018913
(43) Veröffentlichungstag der Anmeldung: 18.12.1991
(73) Patentinhaber: HOECHST AKTIENGESELLSCHAFT, 65926 Frankfurt am Main (DE)
(72) Erfinder: Raab, Klaus, W-8269 Burgkirchen (DE)

(56) Entgegenhaltungen:
- EP-A- 0 194 781
- EP-A- 0 428 039
- THE JOURNAL OF ORGANIC CHEMISTRY, Band 54, 1989, M. HANACK et al, "Facile synthesis of trifluoro- and hexafluoroisopropyl halides", S. 1432-1435
- METHODEN DER ORGANISCHE CHEMIE, Band V/4, 4. Auflage, Georg Thieme Verlag, Stuttgart, DE, Houben-Weyl, "Halogenverbindungen", S. 354-360

## Beschreibung

Die Erfindung betrifft ein Verfahren zur Herstellung von weitgehend fluorierten Alkylbromiden aus weitgehend fluorierten Alkyliodiden.

Es ist aus US-PS 2,678,953 bekannt, Perfluoralkylbromide aus den trockenen Metallsalzen von Perfluoralkylcarbonsäuren durch Umsetzung mit Brom zu gewinnen, wobei die Anwendung von sichtbarem Licht den Umsatz steigert. Im einzigen Beispiel wird das bevorzugte Silbersalz verwendet. Dieses Verfahren ist aufwendig, da erst die Perfluoralkylcarbonsäure erzeugt werden muß, beispielsweise durch die bekannte Umsetzung von Perfluoralkyliodid mit SO₃ oder rauchender Schwefelsäure. Die Säure muß dann in das Metallsalz umgewandelt und dieses getrocknet werden. Ferner erfordert die Umsetzung mit dem giftigen und ätzenden Brom besondere Vorsicht und einen erhöhten apparativen Aufwand (Sicherheitsvorkehrungen, Korrosion). Außerdem geht bei der Reaktionskette eine CF₂-Gruppe des Perfluoralkyliodids verloren.

Es ist ferner aus US-PS 2,875,253 bekannt, einen mit Fluor, Brom und gegebenenfalls mit Chlor substituierten, niedrigen Kohlenwasserstoff als Telogen mit einem Fluor enthaltenden Olefin, das zusätzlich Chloratome enthalten kann, in Gegenwart eines peroxidischen Polymerisations-Promotors zu telomerisieren. Als mögliche Telogene sind unter anderem CF₃Br, CF₂BrCl, CF₂Br₂, C₂F₅Br, C₂F₄BrCl, C₃F₆BrH und C₃F₆Br₂ genannt, unter einer Vielzahl möglicher Fluor enthaltender Olefine Tetrafluorethylen. Die Umsetzung dieser Verbindungen müßte zu weitgehend fluorierten Alkylbromiden im Sinne der vorliegenden Erfindung führen, jedoch ist kein Beispiel vorhanden, aus dem zu entnehmen wäre, unter welchen genauen Bedingungen und mit welchem Erfolg die Umsetzung mit Tetrafluorethylen durchführbar ist. In den Beispielen wird als Fluor enthaltendes Olefin immer CF₂=CFCl eingesetzt.

In einem Aufsatz von Long, Higgins, Mattrey, Mitten und Multer über Radiopake Fluorkohlenwasserstoffe (R.E. Banks, "Preparation, Properties and Industrial Applications of Organofluorine Compounds", 1982, Ellis Horwood Ltd. Publishers/Chichester, Seiten 139 bis 156) ist am Ende der Ausführungen (Seite 154 unten) erwähnt, daß das für die Untersuchungen verwendete Perfluor-n-hexylbromid bzw. Perfluor-iso-heptylbromid durch thermische Bromierung der entsprechenden Perfluoralkyliodide mit elementarem Brom erzeugt wurde, doch fehlen nähere Angaben. Zweifellos steigen die weiter oben bereits erwähnten apparativen Schwierigkeiten bei Anwendung von Brom, wenn thermisch, das heißt bei höheren Temperaturen, mit diesem aggressiven Stoff gearbeitet wird.

R. N. Haszeldine, J. Chem. Soc., 1953, Seiten 3 761 bis 3 768 sowie Huang Bingnan und Huang Weiyuan, Shanghai Inst. Org. Chem. Acad. Sinica, Huaxue Xuebao 42, Seiten 1 106 bis 1 108 (C.A. 102; 78312x), 1984 beschreiben die photochemische Bromierung von Perfluoralkyliodiden [Beispiele: R_{f}I oder Cl(CF₂)₄I] mit Brom unter UV-Strahlung. Nach 168 beziehungsweise 50 Stunden Umsetzung wird R_{f}Br oder Cl(CF₂)₄Br in sehr guter Ausbeute erhalten. Allerdings ist auch dieses Verfahren apparativ und energetisch aufwendig.

Schließlich ist aus JP-Kokai 60-184033-A2, 1985 (C.A. 104; 88106p) bekannt, CₙF₂ₙ₊₁Br (n = 6 bis 11) durch Umsetzung von CₙF₂ₙ₊₁I mit Brom in Gegenwart einer Radikale erzeugenden Verbindung (zum Beispiel Azodiisobutyronitril) zu erzeugen. Es wird so C₆F₁₃Br mit 40 % Ausbeute gewonnen. Die Anwendung des elementaren Broms erfordert auch hier apparative Vorkehrungen.

In den deutschen Patentanmeldungen P 39 37 567.6 und P 40 04 783.0 wird vorgeschlagen, weitgehend fluorierte Alkyliodide mit Bromidionen, die als Salze mit bestimmten Kationen vorliegen, in einem dipolaren, aprotischen Lösungsmittel umzusetzen, wobei in Gegenwart bestimmter Metall-Komplex-Verbindungen oder eines Alkalisalzes einer Hydroxyalkansulfinsäure gearbeitet werden kann, um die Ausbeute an Perfluoralkyliodid zu verbessern. Obwohl mit diesen Verfahren nicht die Schwierigkeiten, beispielsweise Korrosion der Apparateteile, auftreten, wie sie beim Arbeiten mit elementarem Brom bei höheren Temperaturen in Kauf genommen werden müssen, benötigen diese Verfahren Lösungsmittel und andere Zusatzstoffe, die die Herstellkosten verteuern und ermöglichen häufig nur Ausbeuten an weitgehend fluoriertem Alkylbromid, die noch zu wünschen übrig lassen.

Aufgabe der Erfindung ist es, ein Verfahren zur Herstellung weitgehend fluorierter Alkylbromide zur Verfügung zu stellen, das gute Ausbeuten ohne Verwendung von Lösungsmitteln, Katalysatoren und ähnlichen Zusatzstoffen ergibt und nicht die Schwierigkeiten aufweist, die das Arbeiten mit elementarem Brom bei höheren Temperaturen mit sich bringt.

Das neue Verfahren zur Herstellung von weitgehend fluorierten Alkylbromiden aus Verbindungen der Formel

X-(CF₂)ₙ-I (I),

in der bedeuten: X = H, F, Cl, Br, I oder (CF₃)₂CF- und n = 2 bis 16, ist dadurch gekennzeichnet, daß 1 mol gebundenes Iodatom in der Verbindung der Formel (I) mit 1 bis 6 mol Bromidionen, die als mindestens ein wasserfreies Salz, ausgewählt aus der Gruppe: Bromide von Metallen der Gruppen I a, I b, II a, II b des Periodensystems der Elemente, CsBr₃, MnBr₂, FeBr₂, FeBr₃, CoBr₂, NiBr₂, SnBr₂, PbBr₂, TlBr, IrBr, IrBr₂, PtBr₂ und PtBr₄, vorliegen, bei 80 bis 450 °C unter Atmosphärendruck oder dem autogenen Druck der Reaktionsmischung ohne Zusatz weiterer Stoffe unter guter Durchmischung umgesetzt wird.

Verbindungen der obengenannten Formel (I) sind nach verschiedenen bekannten Verfahren herstellbar. Beispielsweise kann durch Umsetzung von Iod mit Iodpentafluorid und Tetrafluorethylen Perfluorethyliodid erzeugt werden, das seinerseits durch Telomerisation mit weiterem Tetrafluorethylen zu höheren Perfluoralkyliodiden umgesetzt werden kann. Analoge Verbindungen sind durch Einsatz entsprechender Ausgangsstoffe erhältlich. Teilweise sind Verbindungen der Formel (I) Marktprodukte. Vorzugsweise werden solche Verbindungen der Formel (I) eingesetzt, in denen X = I ist und insbesondere solche, in denen X entweder (CF₃)₂CF- oder F bedeutet. Verbindungen der Formel (I), in denen n größer als 16 ist, ergeben im allgemeinen längere Reaktionszeiten und häufig schlechtere Ausbeuten, sie sind auch in der Regel weniger gut verwendbar. Wegen ihrer guten Anwendbarkeit sind Verbindungen der Formel (I) bevorzugt, in denen n 4 bis 12 und insbesondere 6 bis 8 bedeutet. Es können auch Mischungen von Verbindungen der Formel (I) eingesetzt werden, die unterschiedliche Substituenten X und/oder unterschiedliche Zahlen n aufweisen.

Erfindungsgemäß wird 1 mol gebundenes Iodatom der Verbindung der Formel (I) mit 1 bis 6 mol Bromidionen, die als Salze mit verschiedenen Kationen vorliegen können, umgesetzt. Werden je 1 mol Iodatom in der Verbindung der Formel (I) weniger als 1 mol Bromidion verwendet, werden schlechtere Ausbeuten erhalten. Prinzipiell können über 6 mol Bromidionen je 1 mol Iodatom in der Verbindung der Formel (I) eingesetzt werden, jedoch wird im allgemeinen hierdurch keine Ausbeuteverbesserung beobachtet, so daß dies ein unnötiger Aufwand ist. Vorzugsweise werden je 1 mol Iodatom in der Verbindung der Formel (I) 1,2 bis 3 mol Bromidionen verwendet.

Die Bromidionen sollen als mindestens ein wasserfreies Salz mit einem Metall aus den Gruppen I a, I b, II a, II b des Periodensystems der Elemente, beispielsweise KBr, CuBr₂, MgBr₂, ZnBr₂, ferner als MnBr₂, FeBr₂, FeBr₃, CoBr₂, NiBr₂, SnBr₂, PbBr₂, TlBr, IrBr, IrBr₂, PtBr₂, PtBr₄, vorliegen. Geeignet sind auch komplexe Metallbromide, wie CsBr₃. Gute Ergebnisse werden erhalten, wenn mindestens ein wasserfreies Alkalimetallbromid oder mindestens ein Bromid des ein- oder zweiwertigen Kupfers eingesetzt wird.

Die Metallbromide werden in der Regel als solche eingesetzt; sie können jedoch auch entweder kurz vor oder während der Umsetzung mit den weitgehend fluorierten Alkyliodiden der Formel (I) erzeugt werden, entweder aus einem Metallbromid von niedrigerer Wertigkeit, beispielsweise Caesiummonobromid, Kupfer-(I)-bromid, Eisen-(II)-bromid und Brom, oder dem entsprechenden Metall, beispielsweise Kupfer, Eisen oder Zink und Brom, wobei für letzteres Verfahren die Alkali- und Erdalkali-Metalle wegen ihrer heftigen Reaktion mit Brom weniger geeignet sind. Das Brom wird hierbei zweckmäßig in den zur Erzeugung der mehr Brom enthaltenden Metallsalze notwendigen stöchiometrischen Mengen oder in geringeren Mengen eingesetzt. Ferner können Metallbromide von niedrigerer Wertigkeit aus dem entsprechenden Metall, beispielsweise Kupfer oder Zinn, und dem höherwertigen Metallbromid, beispielsweise Kupfer-(II)-bromid oder Zinn-(IV)-bromid, erzeugt werden.

Es kann für das erfindungsgemäße Verfahren sowohl ein einzelnes Metallbromid als auch eine Mischung verschiedener Metallbromide, die sich sowohl in der Metallkomponente wie auch in deren Wertigkeit beziehungsweise im komplexen Charakter des Bromids unterscheiden können, verwendet werden.

Das Metallbromid oder die Metallbromide wie auch gegebenenfalls das Metall werden zweckmäßig in fein verteilter Form mit großer Oberfläche, beispielsweise pulverisiert, eingesetzt. Während der Umsetzung mit dem weitgehend fluorierten Alkyliodid der Formel (I) wird die Reaktionsmischung gut durchmischt, beispielsweise durch Schütteln, Rühren, Mahlen, Umschaufeln, Kneten und ähnliche bekannte Methoden.

Zur Vermeidung unerwünschter Nebenreaktionen soll das verwendete Metallbromid möglichst wasserfrei sein. Während der Reaktion wird zweckmäßig in einer trockenen Inertgasatmosphäre, beispielsweise Stickstoff oder Argon, gearbeitet. Eine Intensivtrocknung des Metallbromids kann unmittelbar vor der Mischung mit dem weitgehend fluorierten Alkyliodid geschehen, beispielsweise bei Unterdruck und erhöhter Temperatur mit oder ohne Durchleiten beziehungsweise Überleiten von trockenem Inertgas oder trockenem Bromwasserstoffgas.

Die einzusetzenden Ausgangsstoffe werden in der Regel vollständig vor Beginn der Reaktion zusammengegeben; es kann jedoch auch entweder das weitgehend fluorierte Alkyliodid oder das Metallbromid vollständig vorgelegt und ein Teil der nicht vorgelegten Komponente zu Beginn und weitere Teile dieser Komponente im Verlauf der Reaktion zugegeben werden.

Die oben beschriebene erfindungsgemäße Reaktion wird bei einer Temperatur von 80 bis 450 °C durchgeführt, wobei sie zweckmäßig unter normalem Atmosphärendruck oder unter dem autogenen Druck der Reaktionsmischung stattfindet. Die Anwendung höherer Drucke ist in der Regel nicht erforderlich und stellt einen unnötigen Aufwand dar. Unterhalb 80 °C verläuft die Reaktion im allgemeinen zu langsam, oberhalb 450 °C wird vermehrt die Bildung unerwünschter Nebenprodukte festgestellt.

Wenn CsBr₃ oder ein ähnliches komplexes Bromid mit den Anionen Br₃⁻, IBr₂⁻ oder I₂Br⁻, insbesondere komplexes Alkalimetallbromid, verwendet wird, wählt man zweckmäßig eine Temperatur im unteren Teil des oben angegebenen Bereiches, vorteilhaft 100 bis 150 °C. Dasselbe gilt, wenn höherwertige Metallbromide unter Verwendung von Brom während der erfindungsgemäßen Reaktion erzeugt werden. In allen anderen Fällen, wenn also kein CsBr₃, anderes komplexes Bromid oder Brom eingesetzt wird, wird zweckmäßig eine Temperatur im mittleren oder oberen Teil des angegebenen Temperaturbereiches gewählt. Vorteilhaft wird bei 220 bis 350 °C gearbeitet.

Die Reaktionsdauer hängt von der angewendeten Temperatur, den eingesetzten Ausgangsstoffen und dem gewünschten Umsatz ab und beträgt im allgemeinen 2 bis 80 Stunden; eine längere Reaktionsdauer ist möglich, jedoch wird meist kein zusätzlicher Effekt beobachtet, der die immer schlechter werdenden Raum-Zeit-Ausbeuten rechtfertigen würde. Häufig werden bei einer Reaktionsdauer von 5 bis 20 Stunden gute Ergebnisse erhalten.

Nach Beendigung der Reaktion wird die Reaktionsmischung abgekühlt und entweder gleich destilliert, fraktioniert destilliert oder filtriert und der abfiltrierte Feststoff zur Gewinnung von noch adsorbiertem, weitgehend fluoriertem Alkylbromid gewaschen oder getrocknet oder der Reaktionsmischung zunächst Wasser zugesetzt, die Phase, die die weitgehend fluorierten Alkylbromide enthält, abgetrennt und nun fraktioniert destilliert, wobei, wenn erforderlich, verminderter Druck angewendet wird. Wenn im Destillat Iod - kenntlich durch eine charakteristische Färbung - enthalten ist, kann dieses durch Ausschütteln oder Ausrühren mit verdünnter Natronlauge entfernt werden.

Die nach dem erfindungsgemäßen Verfahren erzeugten, weitgehend fluorierten Alkylbromide können im medizinischen Sektor eingesetzt werden, wie zum Beispiel als Kontrastmittel bei Untersuchungen mit Röntgenstrahlen oder mit Ultraschall, beispielsweise zur Sichtbarmachung von Tumoren, zur Organperfusion sowie in wäßriger Emulsion als Blutersatzstoff. Weitere Anwendungen der weitgehend fluorierten Alkylbromide sind Hochtemperatur-Inertflüssigkeiten und Kontrastmittel für ¹⁹F-Kernresonanz-Spektral-(NMR)-Analyse.

Im Gegensatz zu den bekannten Verfahren ermöglicht es das erfindungsgemäße Verfahren in wenig aufwendigen Apparaturen ohne Schwierigkeiten bezüglich Korrosion und besondere Sicherheitsvorkehrungen zu arbeiten. Im Vergleich zu den neuerdings vorgeschlagenen Verfahren, werden, wie oben bereits ausgeführt, überraschend gute Ausbeuten an weitgehend fluorierten Alkylbromiden erhalten, obwohl auf die Anwendung von Zusatzstoffen, wie Katalysatoren und Lösungsmittel, die das Verfahren teurer macht, verzichtet wird. Sofern bei dem neuen Verfahren Nebenprodukte in nennenswerten Mengen anfallen, können diese auf üblichem Wege isoliert und verschieden verwendet werden. Die Verbindungen, die anstelle des Iodatoms in obengenannter Formel (I) ein Wasserstoffatom oder einen fluorierten Alkylrest tragen können, dienen beispielsweise als Wärmeträgerflüssigkeiten.

Nachfolgende Beispiele sollen die Erfindung näher erläutern.

### Beispiel 1

In ein Bombenrohr von 25 cm³ Inhalt, das aus Glas besteht, werden 12,8 g (0,06 mol) feinzerriebenes Caesiumbromid der Formel CsBr gegeben und bei 200 °C unter einem Druck von 10 Pa 4 Stunden behandelt, um restliches Wasser zu entfernen. Nach dem Abkühlen auf Raumtemperatur werden in das Bombenrohr unter Einleiten von trockenem Stickstoff 21,85 g (0,04 mol) Perfluoroctyliodid der Formel CF₃(CF₂)₇I gegeben, das Bombenrohr zugeschmolzen und 10 Stunden bei 300 °C geschüttelt. Je 1 mol gebundenes Iodatom im Perfluoroctyliodid werden 1,5 mol Bromidionen eingesetzt. Nach Beendigung der Reaktion wird das Bombenrohr gekühlt, geöffnet, die flüssige fluororganische Phase abdekantiert und aus dem zurückbleibenden Feststoff, der im wesentlichen aus Caesiumbromid und Caesiumiodid besteht, adsorbiertes fluororganisches Produkt bei einer Sumpftemperatur bis 250 °C abdestilliert. Das abdestillierte und das dekantierte fluororganische Produkt werden vereinigt. Die so erhaltenen 18,8 g enthalten nach ¹⁹F-NMR-spektroskopischer und gaschromatographischer Analyse 33,9 Gew.-% CF₃(CF₂)₇Br; 62,5 Gew.-% CF₃(CF₂)₇I; 1,7 Gew.-% CF₃(CF₂)₇H und 1,5 Gew.-% CF₃(CF₂)₁₄CF₃. Die Ausbeute an CF₃(CF₂)₇Br, bezogen auf eingesetztes Perfluoroctyliodid, beträgt 31,9 %; 53,8 % des Perfluoroctyliodids wurden zurückgewonnen.

### Beispiel 2

In einen Glaskolben von 250 cm³ Inhalt, der mit Innenthermometer, Rührer, Gaseinleitungsrohr und einem Intensivkühler mit anschließendem, CaCl₂ gefülltem Trockenrohr ausgerüstet ist, werden 140 g eines Produktes gegeben, das zu 80 Gew.-% aus Caesiumtribromid der Formel CsBr₃ und zu 20 Gew.-% Caesiumbromid der Formel CsBr besteht und das aus wasserfreiem Caesiummonobromid und Brom in exothermer Reaktion hergestellt wurde. Es werden 0,3 mol Caesiumtribromid und 0,13 mol Caesiummonobromid eingesetzt. Unter Einleiten von trockenem Stickstoff werden in den Glaskolben 109,2 g (0,2 mol) Perfluoroctyliodid gegeben und die Mischung im Kolben während 16 Stunden unter normalem Luftdruck bei Rückflußtemperatur (100 bis 130 °C) gerührt. Auf 1 mol gebundenes Iodatom werden 5,15 mol Bromidionen eingesetzt. Nach Beendigung der Reaktion und Abkühlen auf Raumtemperatur wird der Kolbeninhalt unter Anwendung von Unterdruck filtriert. Der im wesentlichen aus Caesiumsalzen bestehende Filterrückstand wird mit Trichlortrifluorethan gewaschen und erneut vermittels Unterdruck filtriert. Aus dem Filtrat wird das Trichlortrifluorethan abdestilliert und der Rückstand mit dem ersten Filtrat vereinigt. Es werden 67,5 g fluororganisches Produkt erhalten, das nach ¹⁹F-NMR-spektroskopischer Analyse aus 95,6 Gew.-% Perfluoroctylbromid und 4,4 Gew.-% nicht umgesetztem Perfluoroctyliodid besteht.

### Beispiel 3

In einem Schüttelautoklaven aus V4A-Edelstahl von 250 cm³ Inhalt werden 86,1 g (0,6 mol) feingemahlenes Kupferbromid der Formel CuBr gegeben und unter einem Druck von 10 Pa bei 200 °C 3 Stunden behandelt, um restliches Wasser zu entfernen. Nach dem Abkühlen auf Raumtemperatur werden unter Einleiten von trockenem Stickstoff 218,4 g (0,4 mol) Perfluoroctyliodid gegeben, der Autoklav geschlossen und während 10 Stunden bei 250 °C unter dem autogenen Druck der Reaktionsmischung von 0,8 MPa geschüttelt. Je mol gebundenes Iodatom werden 1,5 mol Bromidionen eingesetzt. Nach Beendigung der Reaktion wird der Autoklav abgekühlt und sein Inhalt über ein Faltenfilter abfiltriert. Das als Filterrückstand erhaltene feuchte Gemisch aus Kupfersalzen wird in einem Destillationskolben bei einem Druck von 10⁴ Pa bis zu 150 °C Sumpftemperatur erhitzt; es werden durch Kondensation der flüchtigen fluororganischen Produkte 23,6 g Destillat erhalten, das mit den 166,6 g ursprünglichen Filtrat vereinigt wird. Dieses Produkt enthält nach ¹⁹F-NMR-spektroskopischer und gaschromatographischer Analyse 43,3 Gew.-% Perfluoroctylbromid; 54,4 Gew.-% nicht umgesetztes Perfluoroctyliodid und 1,3 Gew.-% CF₃(CF₂)₇H. Die Ausbeute an Perfluoroctylbromid, bezogen auf eingesetztes Perfluoroctyliodid, beträgt 41,3 %.

### Beispiel 4

In ein Bombenrohr von 25 cm³ Inhalt, das aus Glas besteht, werden 13,0 g (0,09 mol) feingemahlenes Kupferbromid der Formel CuBr gegeben und bei 200 °C unter einem Druck von 10 Pa 4 Stunden behandelt, um restliches Wasser zu entfernen. Nach Abkühlen auf Raumtemperatur wird in das Bombenrohr trockener Stickstoff eingeleitet und 16,4 g (0,03 mol) Perfluoroctyliodid gegeben, das Bombenrohr zugeschmolzen und 50 Stunden bei 240 bis 250 °C geschüttelt. Je mol gebundenes Iodatom werden 3 mol Bromidionen eingesetzt. Nach Beendigung der Reaktion wird das Bombenrohr geöffnet und die fluororganischen Produkte durch Erhitzen auf 200 °C aus dem Bombenrohr herausdestilliert. Es werden 12,8 g Destillat erhalten, das nach gaschromatographischer Analyse aus 97,7 Gew.-% Perfluoroctylbromid; 1,9 Gew.-% nicht umgesetztem Perfluoroctyliodid und 0,3 Gew.-% CF₃(CF₂)₇H besteht. Das entspricht einer Ausbeute an 83,5 % Perfluoroctylbromid, bezogen auf eingesetztes Perfluoroctyliodid.

### Beispiel 5

Es wird gearbeitet wie in Beispiel 4 angegeben, jedoch werden anstelle von 13,0 g nur 8,6 g (0,06 mol) Kupferbromid der Formel CuBr und anstelle von 16,5 g 21,85 g (0,04 mol) Perfluoroctyliodid eingesetzt. Je 1 mol gebundenes Iodatom werden 1,5 mol Bromidionen angewendet. Das Bombenrohr wird 10 Stunden bei 300 °C geschüttelt. Bei der anschließenden Destillation, wie in Beispiel 4 beschrieben, werden 18,1 g Destillat erhalten, das nach ¹⁹F-NMR-spektroskopischer und gaschromatographischer Analyse 94,5 Gew.-% Perfluoroctylbromid; 4,1 Gew.-% nicht umgesetztes Perfluoroctyliodid und 0,7 Gew.-% CF₃(CF₂)₇H enthält. Die Ausbeute an Perfluoroctylbromid, bezogen auf eingesetztes Perfluoroctyliodid, beträgt 85,7 %.

### Beispiel 6

Es wird gearbeitet wie in Beispiel 4 angegeben, jedoch wird das Bombenrohr anstelle 50 Stunden bei 240 bis 250 °C 10 Stunden bei 300 °C geschüttelt. Es werden 13,5 g Destillat erhalten, das nach gaschromatographischer Analyse wie folgt zusammengesetzt ist: 97,9 Gew.-% Perfluoroctylbromid; 1,2 Gew.-% nicht umgesetztes Perfluoroctyliodid und 0,4 Gew.-% CF₃(CF₂)₇H. Die Ausbeute an Perfluoroctylbromid, bezogen auf eingesetztes Perfluoroctyliodid, beträgt 88,3 %.

### Beispiel 7

In ein Bombenrohr von 25 cm³ Inhalt, das aus Glas besteht, werden 6,7 g (0,03 mol) Kupferbromid der Formel CuBr₂ "wasserfrei" und 21,85 g (0,04 mol) Perfluoroctyliodid unter Einleiten von trockenem Stickstoff gegeben. Auf 1 mol gebundenes Iodatom werden 1,5 mol Bromidionen eingesetzt. Das Bombenrohr wird zugeschmolzen und 10 Stunden bei 300 °C geschüttelt, dann abgekühlt, geöffnet und der Inhalt destilliert, wie in Beispiel 4 beschrieben. Das Destillat wird mit verdünnter Natronlauge verrührt, um das entstandene Iod zu entfernen, dann von der Natronlauge getrennt und getrocknet. Es werden 17,1 g fluororganische Verbindungen erhalten, die nach gaschromatographischer Analyse folgende Zusammensetzung aufweisen: 95,7 Gew.-% Perfluoroctylbromid; 0,6 Gew.-% nicht umgesetztes Perfluoroctyliodid und 3,3 Gew.-% CF₃(CF₂)₇H. Der vergleichsweise hohe Gehalt an letzterer Verbindung deutet darauf hin, daß der Wassergehalt des als "wasserfrei" gekauften CuBr₂ nicht so niedrig war, wie es für die erfindungsgemäße Umsetzung wünschenswert ist.

### Beispiel 8

In ein Bombenrohr von 25 cm³ Inhalt, das aus Glas besteht, werden 5,5 g (0,03 mol) Magnesiumbromid der Formel MgBr₂ bei 200 °C unter einem Druck von 50 Pa 3 Stunden behandelt, um es zu trocknen. Nach dem Abkühlen auf Raumtemperatur werden unter Einleiten von trockenem Stickstoff 17,8 g (0,04 mol) Perfluorhexyliodid der Formel CF₃(CF₂)₅I in das Bombenrohr gegeben, abgeschmolzen und 10 Stunden bei 300 °C geschüttelt. Je 1 mol gebundenes Iodatom werden 1,5 mol Bromidionen angewendet. Nach Beendigung der Reaktion wird das Bombenrohr auf Raumtemperatur abgekühlt, geöffnet und aus dem Inhalt bis zu einer Sumpftemperatur von 200 °C die flüchtigen fluororganischen Stoffe abdestilliert. Das Destillat wird zur Entfernung geringer Mengen Iod mit verdünnter Natronlauge behandelt, mit Wasser nachgewaschen, die wäßrige Phase abgetrennt und die fluororganische Phase getrocknet. Von letzterer werden 13,3 g erhalten, die nach gaschromatographischer Analyse aus 76,3 Gew.-% Perfluorhexylbromid; 0,9 Gew.-% nicht umgesetztem Perfluorhexyliodid und 21,2 Gew.-% einer Verbindung der Formel CF₃(CF₂)₅H besteht. Die Ausbeute an Perfluorhexylbromid, bezogen auf eingesetztes Perfluorhexyliodid beträgt 63,6 %. Der vergleichsweise hohe Anteil an wasserstoffhaltiger Verbindung deutet darauf hin, daß das Magnesiumbromid noch wasserhaltig war. Ein besser getrocknetes Magnesiumbromid müßte einen deutlich geringeren Anteil an wasserstoffhaltiger Verbindung zugunsten einer verbesserten Ausbeute an Perfluorhexylbromid ergeben.

### Beispiel 9

Es wird gearbeitet wie in Beispiel 8 beschrieben, wobei anstelle von 5,5 g Magnesiumbromid 11,3 g (0,06 mol) Silberbromid der Formel AgBr eingesetzt werden. Nach Aufarbeitung, wie in Beispiel 8 beschrieben, werden 14,5 g fluororganische Flüssigkeit erhalten, die nach gaschromatographischer Untersuchung aus 93,1 Gew.-% Perfluorhexylbromid; 4,2 Gew.-% nicht umgesetztem Perfluorhexyliodid und 2,4 Gew.-% einer Verbindung der Formel CF₃(CF₂)₅H bestehen. Die Ausbeute an Perfluorhexylbromid, bezogen auf eingesetztes Perfluorhexyliodid, beträgt 84,6 %.

## Patentansprüche

1. Verfahren zur Herstellung von weitgehend fluorierten Alkylbromiden aus Verbindungen der Formel
X-(CF₂)ₙ-I (I),
in der bedeuten: X = H, F, Cl, Br, I oder (CF₃)₂CF- und n = 2 bis 16, dadurch gekennzeichnet, daß 1 mol gebundenes Iodatom in der Verbindung der Formel (I) mit 1 bis 6 mol Bromidionen, die als mindestens ein wasserfreies Salz, ausgewählt aus der Gruppe: Bromide von Metallen der Gruppen I a, I b, II a, II b des Periodensystems der Elemente, CsBr₃, MnBr₂, FeBr₂, FeBr₃, CoBr₂, NiBr₂, SnBr₂, PbBr₂, TlBr, IrBr, IrBr₂, PtBr₂ und PtBr₄, vorliegen, bei 80 bis 450 °C unter Atmosphärendruck oder dem autogenen Druck der Reaktionsmischung ohne Zusatz weiterer Stoffe unter guter Durchmischung umgesetzt wird.

2. Verfahren nach Anspruch 1, dadurch gekennzeichnet, daß die Umsetzung bei 220 bis 350 °C ohne Anwendung von CsBr₃ durchgeführt wird.

3. Verfahren nach Anspruch 1 oder 2, dadurch gekennzeichnet, daß mindestens ein wasserfreies Alkalimetallbromid eingesetzt wird.

4. Verfahren nach Anspruch 1 oder 2, dadurch gekennzeichnet, daß ein Kupferbromid eingesetzt wird.

5. Verfahren nach Anspruch 1, dadurch gekennzeichnet, daß die Umsetzung bei 100 bis 150 °C in Gegenwart von CsBr₃ durchgeführt wird.

6. Verfahren nach einem oder mehreren der Ansprüche 1 bis 5, dadurch gekennzeichnet, daß mindestens eine Verbindung der Formel (I) eingesetzt wird, in der X = I ist.

7. Verfahren nach einem oder mehreren der Ansprüche 1 bis 5, dadurch gekennzeichnet, daß mindestens eine Verbindung der Formel (I) eingesetzt wird, in der X = F oder (CF₃)₂CF- bedeutet.

8. Verfahren nach einem oder mehreren der Ansprüche 1 bis 7, dadurch gekennzeichnet, daß eine Verbindung der Formel (I) eingesetzt wird, in der n = 4 bis 12 ist.

## Claims

1. A process for the preparation of substantially fluorinated alkyl bromides from compounds of the formula
X-(CF₂)ₙ-I (I)
in which X is H, F, Cl, Br, I or (CF₃)₂CF- and n is 2 to 16, which comprises reacting, with good mixing, 1 mol of bound iodine atom in the compound of the formula (I) with 1 to 6 mol of bromide ions which are present in the form of at least one anhydrous salt selected from the group composed of: bromides of metals of groups I a, I b, II a or II b of the periodic table of the elements, CsBr₃, MnBr₂, FeBr₂, FeBr₃, CoBr₂, NiBr₂, SnBr₂, PbBr₂, TlBr, IrBr, IrBr₂, PtBr₂ and PtBr₄, at 80 to 450°C under atmospheric pressure or the autogenous pressure of the reaction mixture, without the addition of other substances.

2. The process as claimed in claim 1, wherein the reaction is carried out at 220 to 350°C without the use of CsBr₃.

3. The process as claimed in claim 1 or 2, wherein at least one anhydrous alkali metal bromide is employed.

4. The process as claimed in claim 1 or 2, wherein a copper bromide is employed.

5. The process as claimed in claim 1, wherein the reaction is carried out at 100 to 150°C in the presence of CsBr₃.

6. The process as claimed in one or more of claims 1 to 5, wherein at least one compound of the formula (I) in which X is I is employed.

7. The process as claimed in one or more of claims 1 to 5, wherein at least one compound of the formula (I) in which X is F or (CF₃)₂CF- is employed.

8. The process as claimed in one or more of claims 1 to 7, wherein a compound of the formula (I) in which n is 4 to 12 is employed.

## Revendications

1. Procédé pour la préparation de bromures d'alkyle fortement fluorés répondant à la formule :
X - (CF₂)ₙ-I (I)
dans laquelle les significations sont les suivantes : X = H, F, Cl, Br, I ou (CF₃)₂CF- et n = 2 à 16, ce procédé étant **caractérisé** en ce que l'on fait réagir 1 mole d'atome d'iode lié dans le composé de formule (I) sur 1 à 6 moles d'ions bromure, qui se présentent au moins sous la forme d'un sel anhydre, choisi dans le groupe suivant : bromures des métaux des groupes Ia, Ib, IIa, IIb, du tableau périodique des éléments, et parmi CsBr₃, MnBr₂, FeBr₂, FeBr₃, CoBr₂, NiBr₂, SnBr₂, PbBr₂, TlBr, IrBr, IrBr₂, PtBr₂ et PtBr₄, ladite réaction étant effectuée à une température comprise entre 80 et 450°C, sous la pression atmosphérique ou sous la pression autogène du mélange réactionnel, sans addition d'autres produits, et sous une agitation efficace.

2. Procédé selon la revendication 1, **caractérisé** en ce que la réaction est effectuée à une température de 220 à 350°C, sans utilisation de CsBr₃.

3. Procédé selon la revendication 1 ou 2, **caractérisé** en ce que l'on introduit au moins un bromure de métal alcalin anhydre.

4. Procédé selon la revendication 1 ou 2, **caractérisé** en ce que l'on introduit un bromure de cuivre.

5. Procédé selon la revendication 1, **caractérisé** en ce que l'on effectue la réaction à une température comprise entre 100 et 150°C, en présence de CsBr₃.

6. Procédé selon l'une ou plusieurs des revendications 1 à 5, **caractérisé** en ce que l'on introduit au moins un composé de formule (I) dans lequel X = I.

7. Procédé selon l'une ou plusieurs des revendications 1 à 5, **caractérisé** en ce que l'on introduit au moins un composé de formule (I) dans lequel X = F ou représente le groupe (CF₃)₂CF-.

8. Procédé selon l'une ou plusieurs des revendications 1 à 7, **caractérisé** en ce que l'on introduit un composé de formule (I) dans lequel n vaut 4 à 12.
